# EUROPEAN PATENT APPLICATION

(11) **EP 1 302 887 A2**
(43) Date of publication of application: **16.04.2003**
(21) Application number: 02022167.7
(22) Date of filing: 03.10.2002
(51) Int. Cl.: G06F 19/00

(54) **Method and System for identification and indication of deviations in X-ray pictures**

(30) Priority: 03.10.2001 SE 0103283
(71) Applicant: eCare AB, S-115 27 Stockholm (SE)
(72) Inventor: Lundberg, Nina, S-260 42 Mölle (SE)
(74) Representative: Karlsson, Leif Karl Gunnar

(57) **Abstract**

A method and system for systematic identification and indication of deviations between primary and secondary examinations for primary examiners in an X-ray information system (100,200). At least one deviation, which is identified between a primary examination (206), previously stored in a case record database (102) of the system, and a secondary examination performed by a secondary examiner (207), is recorded in the case record database (102). A data processing server (201), operatively connected to the case record database, searches the case record database for identification of deviations and fetches information about identified deviations. The data processing server (201) processes the information and sends a message to the primary examiner (206) in the system, the primary examiner of which at least one deviation has been identified in relation to the examination of the primary examiner (205).

## Description

### Technical Field

The present invention relates to a method and system for systematic identification and indication of deviations between a primary and secondary examination for primary examiners in an X-ray information system.

### Prior Art

Today a lot of resources are put into quality assurance in care, including X-ray treatment. Despite that electronic routines and methods for evaluating the quality of X-ray diagnosis are non-existent. Today, double-checks of internal as well as external X-ray examinations are performed as a matter of routine. Studies show that in spite of this, in 3-18% it exists deviations between initial and final diagnosis within or between X-ray wards. Today, there is no systematic analysis of diagnostic deviations within the X-ray activity. One reason for this is that the present manual method for feedback to previous stages is too heavy and difficult to handle.

Double-checks are performed as a matter of routine by the same examination internally (within an X-ray clinic). Hence, first a radiologist (primary examiner) forms an opinion of the pictures and gives a preliminary response from the examination, then another (commonly more senior) X-ray physician (secondary examiner) checks the same pictures and gives a final opinion of the examination. This two-step process is called a *double-check.*

Each senior radiologist giving a final opinion does always compare its own opinion with the preliminary response. In this process, the secondary Examiner (a more senior radiologist) records, in its own head, if there is a deviation between the two diagnoses. If there is a deviation, this is not recorded in the X-ray information system. Nor is the extent of the deviation recorded in the information system. However, both diagnoses are recorded. Sometimes the secondary examiner can inform the primary examiner about the deviation analogously/verbally. To sum up both diagnoses are recorded in the X-ray information system, but there are no routines for recording of deviations or extent of the deviation between the two diagnoses.

Today, double-checks are performed even as a matter of routine(about 100 000 per year in Sweden) of the same examination *regionally* (between different X-ray units). At regional double examination, the examination is performed and a preliminary response is given at a (smaller) hospital. The patient is then referred to a more specialised hospital (often to a university hospital) which gives a final opinion (diagnosis). In the same way as mentioned above, the secondary examiner records, in its own head, if his diagnosis deviates from the preliminary response given by the first hospital. In that case, there is no record performed of the deviating diagnosis neither in the second nor in the first X-ray information system.

A certain number of medical diagnoses are deviating today. Since these are not recorded in the information system of the medical service, there is no possibility for the radiologist (primary examiner) to get continuous feedback and learn from his mistakes. Nor is there any statistics over the time describing these deviations. Examples of statistics in demand could be the number of deviations related to a certain examination method, the number of deviations related to a certain type of diagnosis etc. Nor is there a possibility for radiologists to get information about its deviations so that they can learn from their mistakes over the time.

In certain X-ray wards in Sweden, manual routines have been set up during terms for information about deviating routines. Information about deviations reach out but with a certain delay and without images and comments. It is a very heavy, cumbersome and costly process and therefore insufficient to manually create methods and routines for feedback to previous steps.

### Summary of the Invention

The object of the present invention is to provide a method for systematically identifying and indicating deviations for primary examiners in an X-ray information system.

The object of the invention is achieved by a method for systematic identification and indication of deviations between a primary and secondary examination for primary examiners in an X-ray information system, wherein at least one deviation, which is identified between a primary examination previously stored in a case record database of the system and a secondary examination performed by a secondary examiner, is recorded in the case record database. A data processing server, operatively connected to the case record database, searches the case record database for identification of deviations and fetches information about identified deviations. The data processing server processes the information and sends a message to the primary examiner in the system, the primary examiners of which at least one deviation has been identified in relation to the examination of the primary examiner.

A more specific object of the invention is to provide a system for systematically identifying and indicating deviations for radiologists (primary examiners).

This object is achieved by a system for systematic identification and identification of deviations between primary and secondary examination for primary examiners in an X-ray information system. The system is characterized by a data processing server configured to search a case record database operatively connected to the server for identification of deviations stored in the case record database, wherein the deviations are deviations, previously stored in the case record database, between a previously stored primary examination and a secondary examination performed by a secondary examiner. The data processing server is further adapted to fetch information about identified deviations from the case record database, process information and send a message to the primary examiner in the system, the examiner of which at least one deviation has been identified in relation to the examination of the primary examiner.

An advantage with a method and system according to the invention is the possibility that information about deviations can reach out to primary examiners fast, flexible, simple and secure in text and image format and give a possibility to meet and exchange experience and knowledge electronically, synchronously or asynchronously. Automatic and continuous deviation reports form the basis for the education, give the physicians the opportunity to increase their competence, identification of problem areas of diagnoses, by recording and analysis of diagnostic deviations. The system and method also give the pre-requisite of reduced expenses.

### Short Description of the Drawings

The invention is described in the following with reference to the accompanying drawings, in which
FIG 1 is a schematic view of a system configuration including an X-ray information system according to the invention,
FIG 2 and FIG 3 show a flow chart of the method for identification and indication of deviations for primary examiners in the X-ray information system of FIG 1, and

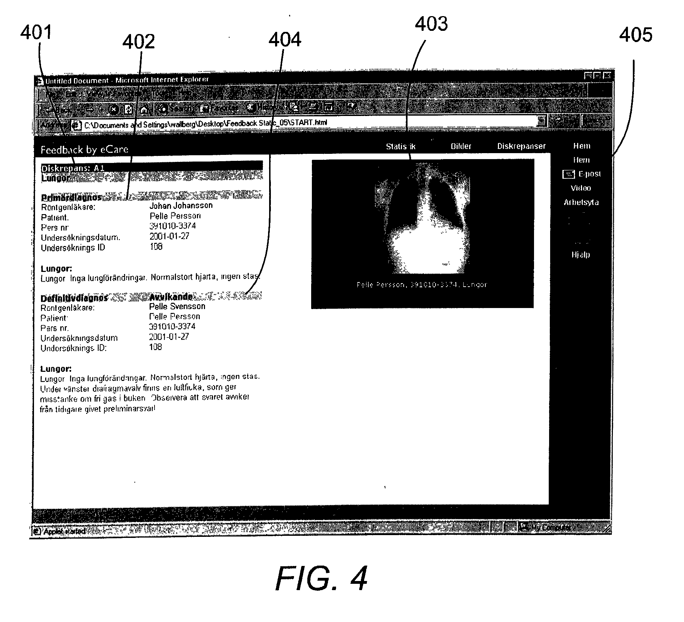

FIG 4 illustrates an example of how a deviation is displayed on a computer screen for a primary examiner at follow-up.

### Detailed Description of the Invention

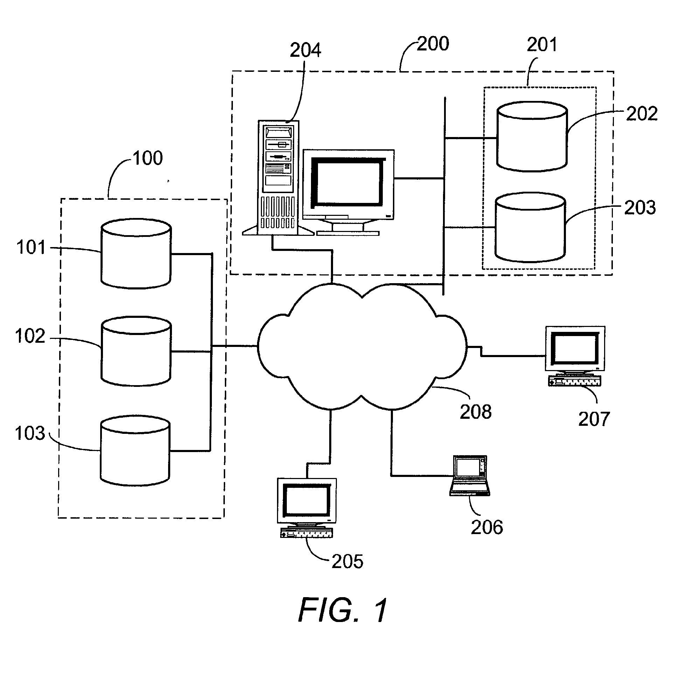

FIG 1 shows an example of a system configuration, including an X-ray information system according to the present invention arranged to systematically identify and indicate deviations between diagnoses for feedback report to radiologists (primary examiners).

The system configuration in FIG 1 comprises, except the X-ray information system 200 according to the invention, described in further detail below, an existing system 100 with an image database (PACS) 101 for storage of X-ray pictures, a first case record database 102 (RIS) for storage of diagnoses or answers recorded by the radiologist (primary examiner) and a second case record database 103 (HIS) for storage of other case record information.

The X-ray information system 200 according to the invention comprises a data processing server 201, including at least an internal database 202 of the X-ray information system 200 for storing data, which are used by the X-ray information system, and a data processing block 203 with tools for processing data stored in the internal database with a method according to the invention for identification and indication of deviations between diagnoses. Additionally, the X-ray information system 200 comprises an application server 204 for accounting and distribution of data and information resulting from the data processing according to the invention. Users of the X-ray information system communicate with the system through one or more terminals or computers 205, 206 and 207 connected to the system. In other embodiments of the network configuration, more or fewer terminals can be connected to the system.

In this embodiment, the application server is a server computer with a processor, internal memory, for example a RAM memory and an external memory such as a hard disc(s) or another external storage medium. In other embodiments, the application server can be another general computer, for example a work station, but is not limited thereto.

Although it seems that the system components, i.e. the data processing system 201, 202, 203, the application server 204 and the terminals 205-207 of the X-ray information system 200, according to the invention as well as the databases 101, 102 and 103 in the existing system, are located near to and is operatively connected to each other in FIG 1, this is only done for the purpose of illustration of the system configuration. It is likewise possible that any, some or every component of the system configuration are located within a shorter or longer distance from each other. The communication between the included components can, within the scope of the invention, be done over a LAN, a WAN, an intranet, the Internet or another electronic or optic communication network or a combination of these networks 208. Such different network configurations are well known and are not described in further detail herein.

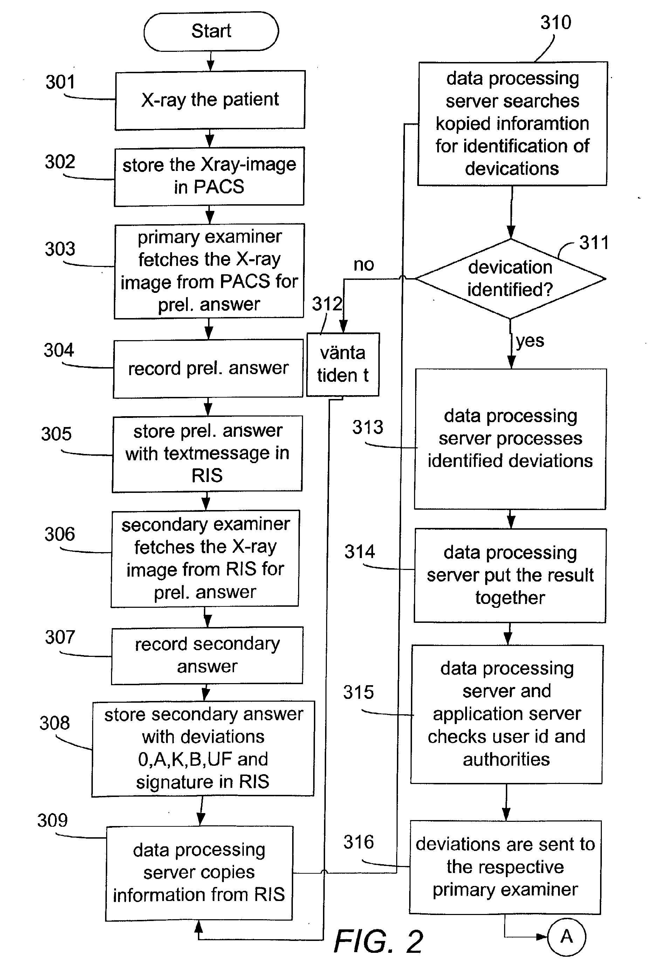

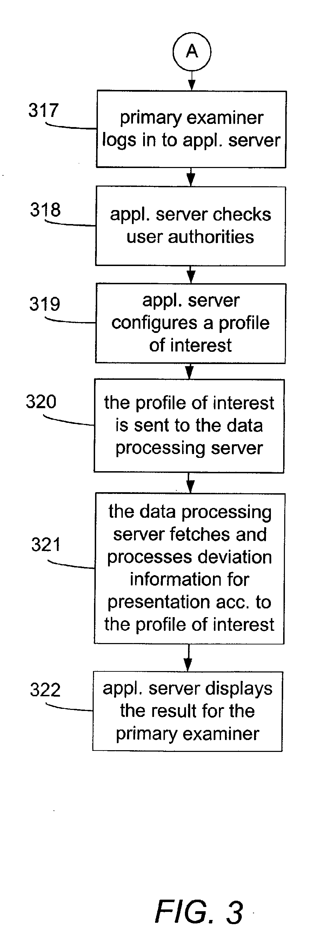

Reference is now made to the flow charts in FIG 2 and FIG 3 on the drawings for description of the method according to the invention for identifying and indicating deviations between diagnoses of X-ray images.

To identify deviations in the system with a method according to the invention, X-ray is performed by for example a nurse in step 301. The X-ray image is stored by the nurse in the first database 101 by means of the terminal 205 connected to the system in step 302 for being put by electronically for a longer or shorter time. In step 303, the X-ray images are fetched from the database through the terminal 206 and is checked by a radiologist (primary examiner), who gives a preliminary answer to the examination. The preliminary answer is recorded in step 304 and is stored with a signature in the form of a text message in the second database 102 in step 305. The images are put by again for a longer or shorter period. Then, the same X-ray image is fetched and checked again but by another (commonly more senior) radiologist (secondary examiner) through a terminal 207 in step 306. The secondary examiner gives a final opinion on the examination, which is recorded in step 307 and stored with a signature in the form of a text message in the second database 102 in step 308.

This two-step process is called a double-check. Each senior radiologist, who gives his final opinion, always compares his own opinion with the preliminary answer. In the method according to the invention, the secondary examiner (a more senior radiologist) analyses whether there is a deviation between the two diagnoses. If there is a deviation, it is/they are recorded in the second database 102 in step 308.

The deviations are classified according to the invention: 0, A, K , B and UF, where 0 means "no deviation" and A, K, B, UF are deviations of more and more significance.
"A" corresponds to a deviation with minor influence for the patient's further investigation, treatment and prognosis.
"K" corresponds to a deviation of moderate significance for the patient's further investigation, treatment and prognosis, for example biliary calculus or inguinal hernia, which have not been observed on the computer tomography performed by another indication.
"B" corresponds to deviations with great significance for the patient's further investigation, treatment and prognosis. An example of such a deviation is emergency conditions or malignity that is misconstrued, for example gas in the abdomen, abdomen or parenchyma haemorrhage, cancer of the colon, pneumothorax, intra or extra cerebral haemorrhage, aorta aneurysm etc.
"UF" corresponds to follow-up cases that the physician for some reason wants to follow up.

The above mentioned levels and what the respective level corresponds to is only examples and the invention is, however, not limited to these five levels. In alternative embodiments of the invention, the deviations can likewise be divided into more or fewer levels and with other names.

With a certain time interval, for example once per one day and night, relevant parts of the information stored in the first and second databases 101, 102 is copied i.e. deviation information such as deviation codes, corresponding primary answers, secondary answers and X-ray images, dates etc. - to the data processing server 201 by the data processing block 203 in step 309. The data processing server 201 scans or searches the copied information in step 310 for identification of deviations. If no deviation has been recorded in step 311, the system waits for a specific time t in step 312 before relevant parts of the first and second databases are copied and searched again.

If deviations have been identified, the copied information is stored and indexed to be searchable in an efficient way in the internal database 202 for storage of data, which are used by the X-ray information system, in step 312. Preferably, the information concerning all recorded diagnoses are transmitted.

The data processing server 202 processes the results according to determined algorithms in step 314 and put together the result from the data processing in step 314. The data processing server 202 and the application server 204 separately check the user identity and authorities in step 315, wherein the application server then in step 316 automatically sends information that new deviations have been identified further to the primary examiner, who has performed the preliminary examination. The information that deviations have been identified is sent via one or more networks connecting the application server and the primary examiner. If the transmission is performed over the Internet, the information can be sent via for example an e-mail message to the e-mail address of the primary examiner.

To be notified about the information on deviations, the primary examiner logs into the X-ray information system 200 on his computer 206, for example over the Internet 208 in step 317. The application server 204 and the data processing server 201 check the client's authorities by way of a security block in the data processing server 203 in step 318. The application server then configures a profile of interests in step 319, which regulates how the users deviations shall be displayed. In step 320, the profile of interests is sent to the data processing server 201. The data processing server fetches and processes the deviation information for storage in the internal database for presentation according to the profile of interest in step 321. The data processing server 201 then processes the result according to determined algorithms in step 323. The application server 202 puts together and presents the result for the user on the user's client computer 206 in step 322. The information on the deviation, i.e. the deviation code etc. is always supplied by an application, for example by way of an encrypted web interface.

One example of the appearance of a deviation report is shown in FIG 3. On the display screen of the primary examiner, a first field 401 is shown, which indicates the current deviation (0, A, K, B, UF). In the current example, it is an A deviation, i.e. a deviation of minor significance for the patient's further investigation, treatment and prognosis. A second field 402 indicates information about the primary examiner and the result from the primary examination. Additionally, an X-ray image 403 is shown, on which the primary examination has been performed. A third field 404 includes information about the secondary examiner and the result from the secondary examination. In the embodiment in FIG 3 the screen image also has a set of buttons 405 for activation of different functions that can be used by the primary examiner. Some examples of such functions are e-mail, video conference system and common work areas for users in the system etc. for communication with for example the secondary examiner.

The method according to the invention is preferably implemented by way of a computer program including program code for the execution of the steps according to the method when the program is run on a computer 200 or a distributed computer system as described above. The computer program is in one embodiment a computer program product including program code stored on a computer readable memory to perform the method according to the invention when the product is run on a computer. The program can be source code, object code, a partially compiled form, or in any other form suitable for use in implementation of the process according to the invention. The carrier can be a disc, a computer memory, a RAM or an electrical signal.

Although the present invention has been described with reference to a preferred embodiment and in conjunction with an existing system with a specific division of information in different database, other divisions of data and information can be done for storage in a common or a number of different databases. Consequently, the invention is not limited to the image database (PACS) for storing X-ray images, the first case record database (RIS) for storing diagnoses or answers recorded by the radiologist (primary examiner) and the second case record data base (HIS) for storing other case record information. For example, the information in the image database and the case record data base may be stored in the same database in another embodiment of the invention. Other embodiments and variations are however likewise possible within the scope of the invention, which is best defined by the accompanying claims.

## Claims

1. A method for systematic identification and indication of deviations between primary and secondary examinations for primary examiners (206) in an X-ray information system (100,200) **characterized by** the steps of:
recording at least one deviation identified between a primary examination previously stored in a case record database (102) of the system and a secondary examination in the case record data base (102) (307,308) performed by a secondary examiner (207),
a data processing server (201) operatively connected to the case record database (102), searching the information in the case record database (102) for identification of a deviation (0,A,K,B,UF) (309,310,312),
the data processing server processing the information (315) with identified deviations (0,A,K,B,UF) and sending a message to the primary examiner (206) in the system, for which at least one deviation has been identified in relation to the primary examiner's examination (316).

2. A method according to claim 1, **characterized in that** the data processing server (201) copies information with interval from the case record database (102) for search and identification of deviations (309,310).

3. A method according to claim 1 or 2, **characterized in that** the primary and secondary examinations are performed on X-ray images stored in an image database (101) of the system.

4. A method according to any of the claims 1-3, **characterized in that** the deviations are classified in different levels for registration.

5. A method according to claim 3, **characterized in that**:
a first level of deviation corresponds to a deviation with minor significance for the patient's further investigation, treatment and prognosis,
a second level of deviation (K) corresponds to a deviation with moderate significance for the patient's further investigation, treatment or prognosis,
a third level of deviation (B) corresponds to a deviation with great significance for the patient's further investigation, treatment or prognosis, and
a fourth level of deviation (UF) corresponds to cases that for some reason requires a follow-up.

6. A system for a systematic identification and indication of deviations between primary and secondary examinations for primary examiners (206) in an X-ray information system (100,200) **characterized by** a data processing server (201,202,203) configured to:
search information stored in a case record database (102) operatively connected to the server (201) for identification of deviations (0,A,K,B,UF) stored in the case record database (102), wherein the deviations are deviations previously stored in the case record database (102) between a previously stored primary examination and a secondary examination performed by a secondary examiner (207), and
process the information and send a message to the primary examiner (206) in the system, the primary examiner of which at least one deviation has been identified in relation to the examination of the primary examiner.

7. A system according to claim 6, **characterized in that**:
the data processing server (201) is configured to copy information with interval from the case record database (102) to an internal database (202) of the X-ray information system (100,200), wherein the server (201) is configured to search the internal database (202) for identifying deviations (0,A,K,B,UF) (309,310).

8. A system according to claim 6 or 7, **characterized in that** the data processing server (201) is operatively connected to terminals (205-207) via at least one network (208) for communication of deviations (0,A,K,B,UF) to the primary examiners (206).

9. A system according to claim 8, **characterized in that** the data processing server (201) is configured to:
communicate deviations (0,A,K,B,UF) through an encrypted web interface over the Internet (208) to the primary examiners (206).

10. A system according to any of the claims 6-9, **characterized in that** the data processing server (201) is operatively connected to an image database (101) with X-ray images, on which the primary and secondary examinations are performed, wherein the data processing server (201) is configured to copy X-ray images associated with the deviations for further transmission to the primary examiners (206).

11. A system according to any of the claims 6-10, **characterized in that** the data processing server (201) is configured to copy information about the primary and secondary examinations from the case record database (102) associated with the deviations for further transmission to the primary examiners (206).

12. A computer program comprising program code means for performing the steps of any one of the claims 1-5 when the program is run on a computer.

13. A computer program product comprising program code means stored on a computer readable medium for performing the method of any of the claims 1-5 when said product is run on a computer.
